(19) Europäisches Patentamt
European Patent Office
Office européen des brevets

(11) Veröffentlichungsnummer: **0 332 137 A2**

(12) # EUROPÄISCHE PATENTANMELDUNG

(21) Anmeldenummer: 89103994.3

(51) Int. Cl.⁴: C07C 143/55

(22) Anmeldetag: 07.03.89

(30) Priorität: 08.03.88 US 165648

(43) Veröffentlichungstag der Anmeldung: 13.09.89 Patentblatt 89/37

(84) Benannte Vertragsstaaten: CH DE ES FR GB IT LI NL

(71) Anmelder: CIBA-GEIGY AG
Klybeckstrasse 141
CH-4002 Basel(CH)

(72) Erfinder: Lund, Richard B.
203 Dogwood Drive
Jackson Alabama 36545(US)
Erfinder: McConnell, Wesley W.
203 LaBonne Drive
Saraland Alabama 36571(US)
Erfinder: Ladd, Sam G.
508 W. Ridgelawn Drive
Mobile Alabama 36608(US)

(74) Vertreter: Patentanwälte TER MEER - MÜLLER - STEINMEISTER
Mauerkircherstrasse 45
D-8000 München 80(DE)

(54) **Ein verbessertes Herstellungsverfahren für 4,4'-Dinitrostilben-2,2'-disulfonsäure und deren Salze.**

(57) Ein Verfahren zur Oxidation von 4-Nitrotoluol-2-sulfonsäure zu 4,4′-Dinitrostilben-2,2′-disulfonsäure dadurch gekennzeichnet, dass man langsam eine Lösung oder Dispersion einer katalytischen Menge eines Alkalimetallhydroxids oder -alkoxids zu einer Lösung eines Alkalimetallsalzes der 4-Nitrotoluol-2-sulfonsäure in Dimethylsulfoxid als Lösungsmittel, in Gegenwart einer katalytischen Menge eines organischen oder anorganischen Uebergangsmetallsalzes, -oxides oder -hydroxides gibt, wobei besagte Lösung mit Sauerstoff gesättigt wird, bis die Oxidation praktisch vollständig ist.

EP 0 332 137 A2

## Ein verbessertes Herstellungsverfahren für 4,4′-Dinitrostilben-2,2′-disulfonsäure und deren Salze

Die vorliegende Erfindung betrifft ein verbessertes Herstellungsverfahren für 4,4′-Dinitrostilben-2,2′-disulfonsäure und deren Salze.

Die Verbindung 4,4′-Dinitrostilben-2,2′-disulfonsäure (DNS) ist ein wichtiges industrielles Zwischenprodukt zur Herstellung vieler optischer Aufheller. Grosse Mengen dieser Verbindung werden jährlich hergestellt. Aus diesem Grunde sind jegliche Verbesserungen des Herstellverfahrens wichtig, die die Wirtschaftlichkeit des Herstellungsverfahrens verbessern durch Erhöhung der Ausbeute oder/und Verminderung der Nebenprodukte.

Verschiedenste Prozesse zur industriellen Herstellung von 4,4′-Dinitrostilben-2,2′-disulfonsäure und deren Salze sind bekannt. Unter anderem auch die oxidative Kondensation von 2 Mol 4 Nitrotoluol-2-sulfonsäure unter wässrig alkalischen Bedingungen. Uebliche Oxidationsmittel sind Sauerstoff (Luft) in Gegenwart eines Katalysators oder Natriumhypochlorit. Die verschiedenen Nachteile dieser wässrigen Verfahren werden ausführlich auf den Seiten 1 und 2 der U.S. 4,719,051 (12. Januar 1988) von Guglielmetti beschrieben. Zum Beispiel müssen solche Verfahren in grosser Verdünnung durchgeführt werden, d.h. ungefähr 5 % Feststoff, wegen der Reaktionswärme und der schlechten Wasserlöslichkeit des eingangs gebildeten Dinitrobenzyl Zwischenproduktes. Ausserdem werden grössere Mengen Nebenprodukt gebildet.

Als ein Resultat mannigfacher Studien oxidativer Kondensationsreaktionen von Arylmethanen unter alkalischen Bedingungen glaubt man das grobe Reaktionsschema und einige der Gründe für die relativ geringen Ausbeuten und hohen Nebenproduktmengen zu kennen. Unter alkalischen Bedingungen existiert p-Nitrotoluol-2-sulfonsäure (HPNTSA) in Form des Salzes, d.h. NaPNTSA wenn die Base Natriumhydroxid ist. In Gegenwart weiterer Base kann das entsprechende Benzylanion im Gleichgewicht mit NaPNTSA stehen. Unter oxidierenden Bedingungen geschieht die Kupplungsreaktion zum wenig löslichen Dinitrodibenzyl Zwischenprodukt (DNDB). Weitere Oxidation wandelt DNDB in die gewünschte DNS um. Unglücklicherweise wird das Reaktionsschema komplexer durch die Bildung unterschiedlicher Mengen von tief gefärbten Stilbenpolyazoverbindungen. Diese Verbindungen entsprechen im allgemeinen der Formel

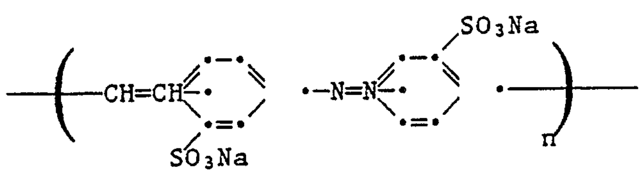

wobei n normalerweise eine Zahl von 1 bis 6 ist. Die Reaktion wird darüberhinaus erschwert durch die Weiteroxidation der DNS. Wenn man die Oxidation lange genug durchführt, so dass fast vollständige Umwandlung der NaPNTSA und die NDB zu DNS erfolgt dann werden grössere Mengen oxidativer Nebenprodukte der DNS gebildet. Der Hauptreaktionsweg zur Bildung und nachfolgenden Zerstörung der DNS kann wie folgt zusammengefasst werden.

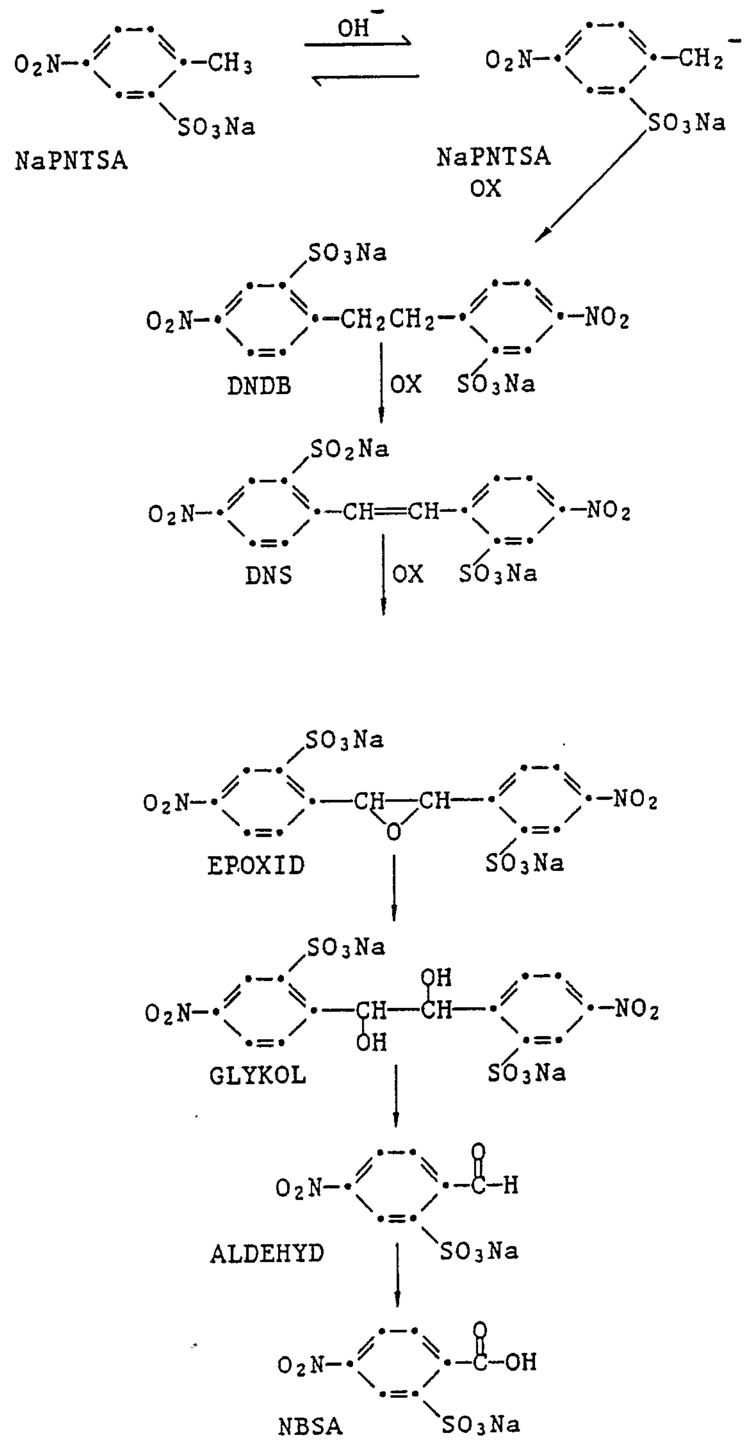

NaPNTSA
NaPNTSA OX
DNDB
DNS
EPOXID
GLYKOL
ALDEHYD
NBSA

Als ein Ergebnis der Bildung von Nebenprodukten und der Weiteroxidation während der DNS-Synthese beträgt die Ausbeute der wässrigen Luftoxidation nur etwa 60-75 % 4,4'-Dinitrostilben-2,2'-disulfonsäure und deren Salze (siehe z.B. DOS 2 258 530) darüberhinaus treten schwerwiegende Abfallprobleme auf.

Jedoch ist bekannt, dass Nitro-, Dinitro- und Trinitrotoluol durch Sauerstoff (Luft) in organischen Lösungsmitteln in Gegenwart starker Basen oxidiert werden kann und in Gegenwart oder Abwesenheit von Katalysatoren kondensiert werden kann, sodass man komplexe Produktmischungen erhält, die die entsprechenden Nitrostilbenverbindungen enthalten (siehe C.A. 84, 58886 n (1976) (Kompolthy et al., Ungarische Patentanmeldung 167,394 veröffentlicht 31. Dezember 1976); Acta Chem. Scand. 25, 3509-3516 (1971); J. Org. Chem. 32, 137-46 (1967) und Advan. Chem. Ser. 51, 112-71 (1965)). Zu beachten ist, dass diese Produkte nicht Nitrostilbensulfonate sind. Weiterhin sind die Ausbeuten dieser Oxidationsreaktionen an Nitrostilbenverbindung gering, weniger als 70 % und werden begleitet durch die Bildung grösserer Mengen

unerwünschter Nebenprodukte.

Obengenannte U.S. 4,719,051 von Guglielmetti offenbart, dass man 4,4′-Dinitrostilben-2,2′-disulfonsäure (DNS) und deren Salze in guter Ausbeute durch die Oxidation von 4-Nitrotoluol-2-sulfonsäure herstellen kann, wenn die Oxidation in einem organischen Lösungsmittel durchgeführt wird.

Dipolare aprotische organische Lösungsmittel werden allgemein als geeignet beschrieben, wobei wie im Beispiel durch Dimethylformamid belegt, die Alkylamide bevorzugt werden. Zusätzlich wird in allen Beispielen, wo eine Ausbeute über 50 % erzielt wird, eine grössere Menge Methanol als zweites Lösungsmittel verwendet. Die Oxidation wird durchgeführt indem man langsam eine Dimethylformamidlösung eines Alkylmetallsalzes von PNTSA, z.B. NaPNTSA zu einer gekühlten Dimethylformamid/Methanolmischung gibt, welche ein Uebergangsmetallsalz als Katalysator enthält, vorzugsweise Mangansulfat und mindestens ein Aequivalent einer Base vorzugsweise ein Alkalimetallhydroxid oder Alkoxid während man gleichzeitig Luft oder Sauerstoff durch die Reaktionslösung leitet, sowohl während der Zugabe als auch in den nachfolgenden Stunden. Durch Befolgen dieser allgemeinen Vorgehensweise werden Ausbeuten von ungefähr 83 % bis zu 95,6 % erhalten.

Obwohl die hohen Ausbeuten dieses Verfahren vorteilhaft erscheinen lassen, im Vergleich zu den wässrigen vorbekannten Verfahren, ist es doch vom Standpunkt einer Kommerzialisierung noch nicht ganz ausgereift. Obwohl die Ausbeute berechnet auf NaPNTSA zufriedenstellend ist, werden doch relativ grosse Mengen zweier verschiedener Lösungsmittel, einer Base und eines Katalysators benötigt. Einer der Gründe für den grossen Ueberschuss von Base ist, dass Dimethylformamid unter alkalischen Bedingungen teilweise umgewandelt wird in ein Formiatsalz und Dimethylamin. Hierdurch wird ein Teil der Base verbraucht, die Menge Dimethylformamid die man zuruckgewinnen könnte reduziert und ausserdem benötigt man eine Säurewäsche um Umweltbelastungen durch Dimethylamin zu verhindern.

Die Rückgewinnung des Dimethylformamids aus dem wässrig-methanolischen Abwasser des Guglielmetti-Prozesses ist schwierig und teuer. Jedoch tritt ein weiteres noch schwerwiegenderes Problem bei der Benutzung von Dimethylformamid auf. Dimethylformamid hat sich im Experiment als teratogen erwiesen und beeinflusst negativ das Zentrale Nervensystem beim Menschen. Hieraus resultieren spezielle Sicherheitsvorkehrungen um weibliches Labor- und Fabrikpersonal vor der Substanz zu schützen, darüberhinaus ist eine Luftüberwachung nötig um zu erreichen, dass für alle Arbeiter die zeitlich gewichtete mittlere Belastung unterhalb 10 ppm ist. Bedingt durch die zusätzlichen Ausgaben für die Arbeitssicherheit sollte der Gebrauch von Dimethylformamid wo dies möglich ist vermieden werden.

## Aufgabe der Erfindung

Es ist eine Aufgabe der vorliegenden Erfindung, ein verbessertes Verfahren zur Herstellung von 4,4′-Dinitrostilben-2,2′-disulfonsäure oder deren Salze zu entwickeln, wobei die Ausbeute und Reinheit hoch ist ohne die Verwendung eines gefährlichen Lösungsmittels, welches die Ueberwachung der Luft am Arbeitsplatz nötig macht und schwierig und teuer zurückzugewinnen ist.

Es ist weiterhin eine Aufgabe dieser Erfindung ein Verfahren zu entwickeln, welches einen deutlich höheren Durchsatz hat als die bekannten Verfahren, dadurch erreichbar, dass man die Konzentration der Reaktanden erhöht und/oder die Reaktionszeit vermindert.

Es ist weiterhin eine Aufgabe dieser Erfindung ein Verfahren zu entwickeln, bei dem die Rückgewinnung des Lösungsmittels in einer einfachen und wirtschaftlichen Art und Weise durchgeführt werden kann.

Es ist weiterhin Aufgabe dieser Erfindung, die Reinigungskosten des wässrigen Abwassers, die mit der Herstellung von DNS verbunden ist zu verringern, erreichbar durch die Verminderung der Menge eingesetzten Schwermetalls, der gefärbten Produkte und/oder oxidativen DNS-Nebenprodukte die dortdrin enthalten sind.

## Die Erfindung

Ueberraschenderweise wurde gefunden, dass die obigen Aufgaben gelöst werden können dadurch, dass man die Oxidation von 4-Nitrotoluol-2-sulfosäure in Form ihrer Alkalimetallsalze mit reinem Sauerstoff oder Luft in Gegenwart von katalytischen Mengen eines Alkalimetallhydroxides oder -alkoxides und eines Uebergangsmetallsalzes in Dimethylsulfoxid (DMSO) als Lösungsmittel durchführt. Vorzugsweise führt man die Oxidation von 4-Nitrotoluol-2-sulfonsäure zu 4,4′-Dinitrostilben-2,2′-disulfonsäure durch, indem man langsam eine Lösung oder Dispersion einer katalytischen Menge eines Alkalimetallhydroxids oder -alkoxids zu einer Lösung eines Alkalimetallsalzes von 4-Nitrotoluol-2-sulfonsäure in Dimethylsulfoxid als Lösungsmittel in Gegenwart einer katalytischen Menge eines organischen oder anorganischen Uebergangsmetallsalzes, -oxides oder -hydroxides gibt, während man gleichzeitig obige Lösung mit Sauerstoff sättigt, bis die Oxidation praktisch vollständig ist.

Dimethylsulfoxid wird als wesentlich weniger gefährliches Lösungsmittel im Hinblick auf die Arbeitsplatzsicherheit angesehen als Dimethylformamid. Es ist keine spezielle Luftüberwachung nötig und die normalen Sicherheitsvorkehrungen bezüglich eines Schutzes vor Kontakt mit Chemikalien sind für den Schutz der Arbeiter ausreichend.

Im Gegensatz zu Dimethylformamid wird Dimethylsulfoxid nicht von Alkalimetallhydroxiden und -alkoxiden angegriffen. Aus diesem Grunde ist im Prinzip eine totale Rückgewinnung des Lösungsmittels möglich.

Während obige Vorteile inherent grössere Sicherheit und Inertheit gegenüber Basen erwartet werden können, gibt es eine Reihe weiterer Vorteile bei der Benutzung von Dimethylsulfoxid als Reaktionsmedium, welche nicht vorhersehbar sind.

Die oxidative Kupplung von PNTSA erfolgt ausgehend von einem Metallsalz von PNTSA und nicht ausgehend von der freien Säure, weil für die Carbanionbildung alkalische Bedingungen erforderlich sind. Die Alkali metallsalze von PNTSA und deren Dibenzylkupplungszwischenprodukte sind löslicher in Dimethylsulfoxid als in Dimethylformamid, wodurch höhere Konzentrationen benutzt werden können. Zusätzlich erfolgt die Oxidationsreaktion schneller in Dimethylsulfoxid und es können höhere Temperaturen in Kauf genommen werden, ohne negative Auswirkungen auf die Reinheit und die Ausbeute zu befürchten. Der Gesamteffekt der sich aus der höheren Konzentration der Reaktanden und der kürzeren Reaktionszeit ergibt ist, dass man ungefähr sechs Mal soviel DNS bei gegebener Reaktorgrösse produzieren kann, wenn man Dimethylsulfoxid als Lösungsmittel verwendet im Vergleich zur Verwendung von Dimethylformamid. Verglichen mit dem in sehr verdünnter wässriger Lösung durchgeführten Verfahren ist der Anstieg des Durchsatzes noch drastischer. Weitere Vorteile werden durch die folgende Diskussion offensichtlich werden.

Das zur Durchführung dieser Erfindung verwendete Lösungsmittel, Dimethylsulfoxid, braucht nicht wasserfrei zu sein aber es ist vorteilhaft wenn der Wassergehalt am Anfang ungefähr 1,5 Gew.% beträgt. Die Menge Wasser kann bis zu 40 % betragen, wenn zum Auflösen der Base ein wasserfreier Alkohol verwendet wird. Wegen der hohen Viskosität der Reaktionslösung ist ein hoher Gehalt an Wasser nicht erwünscht, der eine Durchführung der Reaktion in den bevorzugten Konzentrationsbereich für PNTSA verhindert. Besonders bevorzugt ist ein anfänglicher Wassergehalt der Reaktionsmischung unter 0,5 % insbesondere wenn eine wässrige Lösung des Alkalimetallhydroxids verwendet wird.

Vor der Oxidation wird eine Base zur Neutralisierung der PNTSA benötigt. Bevorzugte Basen sind Lithium-, Natrium- und Kaliumhydroxid. Besonders bevorzugt ist Natriumhydroxid weil es preiswert ist und weil normalerweise die optischen Aufheller, die sich von der DNS ableiten, in Form ihrer Natriumsalze verkauft werden. So ist es von Vorteil während aller Syntheseschritte nur Natriumverbindungen zu benutzen.

Im Patent von Guglielmetti wird empfohlen, die Oxidation von NaPNTSA in Gegenwart eines oder mehrerer Aequivalente einer starken Base durchzuführen. Ueberlicherweise werden etwa 1,6 Moläquivalente dieser starken Base pro Aequivalent NaPNTSA verwendet. Bedingt durch die grosse benötigte Menge Base war es nötig, diese Base in Form des Alkoxids, also z.B. Natriummethoxid einzusetzen oder als praktisch wasserfreies Hydroxid in Methanol gelöst. Wenn eine wässrige Hydroxidlösung in dem hohen molaren Verhältnis des Patentes von Guglielmetti verwendet worden wäre, hätte die grosse Menge Wasser die Reaktion stark beeinträchtigt und die Vorteile des nichtwässrigen Prozesses wären verlorengegangen.

In dem Verfahren dieser Erfindung werden vorzugsweise katalytische Mengen, d.h. 0,05-0,9 Moläquivalente starker Base pro Aequivalent NaPNTSA verwendet. Obwohl man grössere Mengen der Base, wie in der vorbekannten Literatur beschrieben verwenden kann gibt es keinen Vorteil dieses zu tun. Ueberschüssige Mengen von Base können im Gegenteil die Reaktion dadurch verlangsamen, dass sie die Löslichkeit des Dibenzylzwischenproduktes herabsetzen. Zusätzlich erschweren sie die nachträgliche Aufarbeitung, benötigen zur Neutralisierung zusätzliche Säure und belasten das Abwasser unnötigerweise ohne zusätzlichen Nutzen. Bevorzugt ist die Benutzung von nur 0,05-0,4 Moläquivalente Base besonders bevorzugt 0,08-0,2 Moläquivalente; vorteilhaft in einem $C_1$-$C_4$-Alkohol gelöst. Um bei solch geringen Mengen Base reproduzierbare Ergebnisse zu erhalten, müssen selbstverständlich jegliche Quellen zusätzlicher Säure kompensiert werden, so z.B. NaPNTSA, das Uebergangsmetallsalz und Dimethylsulfoxid insbesondere wenn nicht-destilliertes recycliertes Dimethylsulfoxid als Lösungsmittel verwendet wird.

Bevorzugte starke Basen sind die Alkalimetallhydroxide und -alkoxide insbesondere jene von Natrium. Obwohl man eine wasserfreie Base wie z.B. feingemahlenes festes Natriumhydroxid oder ein Alkoxid wie z.B. Natriummethylat in dem hier beschriebenen Verfahren verwenden kann ist es nicht erforderlich eine wasserfreie Base zu verwenden, weil nur sehr geringe Mengen Base benötigt werden. Natriummethylat ist

teuer und ausserdem wird es durch restliche Mengen Wasser in der Reaktionsmischung zum grössten Teil oder vollständig in Natriumhydroxid umgewandelt es sei denn, strengste (und im Falle von Dimethylsulfoxid unnötige) Vorsichtsmassnahmen werden ergriffen um Spuren von Wasser aus der Reaktionsmischung fernzuhalten.

Zur Erleichterung der Handhabung und wegen der Kosten ist es vorteilhaft, die Dimethylsulfoxid-Reaktionsmischung durch Vakuum-Destillation zu trocknen, so dass sie ungefähr 0,3 % Wasser enthält und anschliessend langsam die nötige katalytische Menge Natriumhydroxid zuzugeben in Form festem Natriumhydroxids, welches in einem $C_1$-$C_4$-Alkohol gelöst ist, der kleine Mengen Wasser enthalten kann. So verhindert man die Schwierigkeiten im Umgang und bei der Zugabe kleiner Mengen festem Natriumhydroxids.

Eine weitere bevorzugte Art der Basenzugabe ist 20-50%iges wässriges Natriumhydroxid in einem $C_1$-$C_4$-Alkohol zu lösen, der vorzugsweise wasserfrei ist. In jedem Falle liegt die Base in Form einer einfach zu handhabenden und abzumessenden alkoholischen Lösung vor.

Bevorzugte Alkohole sind Methanol, Ethanol und Isopropanol und deren Mischungen, wobei die Wahl des Alkohols davon abhängig ist, welche Aufarbeitungsmethode benutzt wird. Wenn eine Rückgewinnung des Alkohols nicht gewünscht wird, dann ist Methanol der Alkohol der Wahl, weil er der kostengünstigste ist und weil sich Natriumhydroxid besonders gut in ihm löst. Im Falle einer Rückgewinnung und Recyclierung des Alkohols nach der Kristallisierung der DNS aus der Reaktionsmischung wird vorzugsweise Ethanol (einschliesslich Mischungen mit Methanol und/oder Propanol z.B. SDA-3 oder 94 %iger Alkohol) oder Isopropanol verwendet.

Bevorzugte Mischungsverhältnisse von DMSO zu Alkohol reichen von 50:1 für Methanol zu ungefähr 4:1 für Isopropanol. Obwohl bei der oxidativen Kupplung feuchte Alkohole (bis zu 10 % Wasser im Alkohol) verwendet werden können, wird die Ausbeute verringert und bei steigendem Wassergehalt nimmt die Menge an Azostilbenen und anderen Nebenprodukten zu. Der Gesamtwassergehalt der anfänglichen Reaktionsmasse kann ungefähr 1,5 Gew.% betragen, ohne dass die Ausbeute an DNS signifikant beeinflusst wird oder das grosse Mengen von Nebenprodukten gebildet werden.

Da Alkohole, was auch für Methanol gilt, die Löslichkeit in DMSO von NaPNTSA und insbesondere des Dibenzylzwischenproduktes herabsetzen, sollte der Gebrauch von grossen Mengen vermieden werden. Im allgemeinen sollte mit der minimalen Menge, die benötigt wird um die Base zu lösen, gearbeitet werden.

Eine weitere bevorzugte Art die nötige Basenmenge zuzugeben ist es, die Dimethylsulfoxidmischung durch Vakuumdestillation bis zu einem Wassergehalt von unter ungefähr 0,3 % zu trocknen und dann dazu langsam eine 10-50%ige wässrige Natriumhydroxidlösung zu geben. Da hierbei kein Alkohol verwendet wird, ist die nachfolgende Lösungsmittelrückgewinnung einfacher. Wenn man keinen Alkohol benutzt, muss man für intensives Rühren sorgen, damit eine feine Dispersion von Natriumhydroxid in der Dimethylsulfoxidreaktionsmischung erhalten wird. Vorzugsweise gibt man die Natriumhydroxidlösung maximal 30%ig zu und besonders bevorzugt als 15-20%ige Lösung, um eine gute Dispersion zu ermöglichen. Ist kein Alkohol zugegen benutzt man vorzugsweise 0,2-0,7 Moläquivalente Base.

Der mögliche Temperaturbereich zur Durchführung der Reaktion reicht von wenig über dem Gefrierpunkt der Reaktionsmischung, üblicherweise ungefähr 8 °C, wenn kein Alkohol zu der Mischung gegeben wird bis ungefähr 60 °C. Bevorzugt sind Temperaturen von 10-25 °C und besonders von 12-20 °C. Die Reaktion ist exotherm und es ist einfacher die Reaktionswärme abzuleiten, wenn die Temperatur etwa 4° oder mehr oberhalb des Schmelzpunktes der Reaktionsmischung gehalten wird.

Innerhalb des bevorzugten Temperaturbereiches ist die Reaktion 50-80 Minuten nachdem alle nichtgasförmigen Reaktanden zusammengegeben wurden vollständig, falls man einen $C_1$-$C_4$-Alkohol benutzt um die Base zu lösen. Benutzt man eine wässrige Lösung der Base, ist die Reaktionsdauer 2-3 mal so lang.

Mögliche Oxidationsmittel sind reiner Sauerstoff oder dessen Mischungen mit inerten Gasen, wie z.B. Stickstoff. Aus wirtschaftlichen Gründen verwendet man vorzugsweise trockene Luft. Ein ausreichender Sauerstoff(Luft-) strom und intensive Durchmischung sind die Voraussetzung um hohe Ausbeuten in einer kurzen Reaktionszeit zu erhalten.

Die Oxidation verläuft bei Normaldruck zufriedenstellend, jedoch kann man, um die Löslichkeit des Sauerstoffs im Reaktionsmedium zu erhöhen, bei erhöhten Drucken arbeiten. Da das Anhalten der Luftzufuhr die Oxidation schnell zum Erliegen bringt, ist dies ein Anzeichen, dass die Lösung von Sauerstoff in der Reaktionsmischung der geschwindigkeitsbestimmende Schritt ist. Vom Sicherheitsstandpunkt her gesehen ist dies ein Vorteil.

Die Verwendung anderer Oxidationsmittel wie z.B. Wasserstoffperoxid, wasserfreie Hypochlorite oder Chinone, kann nicht empfohlen werden, wegen der zu befürchtenden weiteren Oxidation der DNS und/oder des Lösungsmittels Dimethylsulfoxid.

Damit die oxidative Kupplung mit Hilfe von Luft oder gasförmigem Sauerstoff mit einer akzeptablen

Geschwindigkeit erfolgt, benötigt man einen Uebergangsmetallkatalysator. Geeignete Katalysatoren sind anorganische Salze, Oxide oder Hydroxide der Uebergangsmetalle und/oder organische Uebergangsmetallverbindungen oder Komplexe, z.B. von Co, Mn, Cr, Ce, Fe, Ni, Cu, Ru, Pd, Pt oder Ir (siehe Homogenous Catalysis of Metal Complexes, Vol. I Chapter 2: Activation of molecular oxygen, pg. 79; Academic Press N.Y. and London, 1974). Wichtige Katalysatoren sind die organischen und anorganischen Salze von Kupfer und Eisen, wie z.B. $CuSO_4 \cdot 5H_2O$ und $FeSO_4 \cdot 7H_2O$. Die Benutzung von Kupferverbindungen sollte jedoch aus ökologischen Gründen vermieden werden, wo es gangbare Alternativen gibt und der Gebrauch von Eisenverbindungen kann bei basischen Reaktionsbedingungen zu Filtrierproblemen führen. Bevorzugte Katalysatoren sind wasserfreie oder hydratisierte Salze, Oxide oder Hydroxide des Mangans und/oder organische Manganverbindungen; wie z.B. Mangansulfat, -hydroxid oder -acetate. Der besonders bevorzugte Katalysator ist $Mn(OAc)_2 \cdot 4H_2O$.

Die Menge des Katalysators kann in weiten Grenzen schwanken. So können Spuren bis hin zu Mengen im Bereich von 0,1-10 Gew.% gerechnet auf 4-Nitrotoluol-2-sulfonsäure verwendet werden. Vorzugsweise arbeitet man mit 0,3-1 Gew.%.

Die Ausgangsverbindung, 4-Nitrotoluol-2-sulfonsäure (HPNTSA) wird in an sich bekannter Weise durch Sulfonierung von 4-Nitrotoluol mit Oleum, z.B. mit 25%igem Oleum, hergestellt. Die Reaktion wird beendet durch die Verdünnung der Sulfonierungsreaktionsmasse mit Wasser oder vorzugsweise mit einer wässrigen $Na_2SO_4$-Lösung. Es ist ein Merkmal der hier beschriebenen Erfindung, dass diese lagerstabile Roh-HPNTSA-Lösung, die üblicherweise 32-36 % HPNTSA, kleinere Mengen verschiedener Nebenprodukte und 3-6 % Schwefelsäure enthält, ohne weitere Reinigung eingesetzt werden kann zur Herstellung von NaPNTSA.

## Ausführliche Beschreibung

### A. Herstellung von NaPNTSA

Die Umwandlung von 4-Nitrotoluol-2-sulfonsäure (HPNTSA) in ihr Natriumsalz (NaPNTSA) ist in der Hauptsache eine Neutralisierung der Sulfosäuregruppen. Jedoch muss dieser Schritt mit Sorgfalt durchgeführt werden, um Bedingungen zu vermeiden, die zur Bildung von intensiv gefärbten Polyazostilbenen führen.

Die bevorzugten Neutralisierungsreagenzien sind $Na_2CO_3$ und NaOH. Die Benutzung von $Na_2CO_3$ verhindert die Bildung von Polyazoverbindungen, aber man benötigt wegen des Aufschäumens während der Neutralisierung, bedingt durch die $CO_2$-Entwicklung und/oder des Auskristallisierens der Reaktanden aus der Lösung, sehr lange Reaktionszeiten. Die Verwendung von konzentrierten NaOH-Lösungen bei hohen Temperaturen führt häufig zu Polyazostilbennebenprodukten.

Wässrige bis zu 50%ige NaOH-Lösungen können verwendet werden um 10-30%ige wässrige Lösungen von Roh-HPNTSA/$H_2SO_4$ ohne die Bildung von Polyazostilbenen zu neutralisieren unter der Voraussetzung, dass der pH-Wert unterhalb 7 gehalten wird und dass die NaOH-Zugabe langsam unter intensiver Vermischung erfolgt, um örtliche hohe Hydroxidkonzentrationen zu vermeiden. Vorzugsweise wird die Temperatur oberhalb 60° gehalten, um übermässige Kristallisation während der Neutralisierung zu vermeiden.

Beim Abkühlen trennt sich der grösste Teil des neutralisierten Natriumsalzes von p-Nitrotoluolsulfonsäure (NaPNTSA) durch Kristallisation vom Nebenprodukt $Na_2SO_4$. Der $Na_2SO_4$-Gehalt nach der Neutralisierung wirkt sich günstig aus, da er die Löslichkeit von NaPNTSA stark herabsetzt. Die Löslichkeit von NaPNTSA in reinem Wasser beträgt bei 26° C ungefähr 13 Gew.%. Bei 12 oder 20 % $Na_2SO_4$ im Wasser geht die Löslichkeit von NaPNTSA bei 26° C auf 1,0 bzw. 0,3 Gew.% zurück. Durch Rückführung eines Teiles des Filtrates in nachfolgende Neutralisierungsschritte kann eine Konzentration von 20 Gew.% $Na_2SO_4$ nach der Neutralisierung und Isolierung aufrecht erhalten werden. Zusätzlich verringert die Rückführung dieses Filtrates, da es noch einwenig gelöstes NaPNTSA enthält, die Verluste an NaPNTSA.

Die Isolierung der kristallisierten NaPNTSA durch Filtration entfernt den grössten Teil des Wassers. Nach der Filtration kann der feuchte Filterkuchen entweder im Vakuum bei Temperaturen von ungefähr 60° getrocknet werden um das restliche Wasser zu entfernen, oder aber vorzugsweise in frisches oder aufgearbeitetes Dimethylsulfoxid gegeben werden und, um das restliche Wasser zu entfernen, einer Vakuumdestillation unterworfen werden. Die Entfernung grosser Mengen Wasser durch Vakuumdestillation von Dimethylsulfoxid ist teuer. Jedoch werden die Kosten des Trocknens durch Vakuumdestillation stark herabgesetzt, dadurch, dass man zuerst die Hauptmenge des unerwünschten Wassers aus der NaPNTSA

durch eine einfache Filtration entfernt.

Die Hauptmenge von $Na_2SO_4$ in dem feuchten Filterkuchen der NaPNTSA wird durch die nachfolgende Filtration der getrockneten Dimethylsulfoxidlösung entfernt. Da die Löslichkeit von NaPNTSA in Dimethylsulfoxid ungefähr 41 Gew.% bei 26 °C beträgt, treten keine Löslichkeitsprobleme während des Trocknungsschrittes auf.

B. Oxidation der NaPNTSA

Gibt man eine getrocknete filtrierte Dimethylsulfoxidlösung von NaPNTSA langsam zu einer Reaktionsmischung bestehend aus Dimethylsulfoxid, einem Uebergangsmetallkatalysator, einer Base und gegebenenfalls einer geringen Menge $C_1$-$C_4$-Alkohols, währenddessen man gleichzeitig Luft durch die Mischung leitet (Reihenfolge der Zugabe der Reaktanden wie im Patent von Guglielmetti) erhält man Ausbeuten von 90-95 %. Geht man jedoch in der umgekehrten Reihenfolge vor und gibt die Base gelöst in Wasser oder einer kleinen Menge eines $C_1$-$C_4$-Alkohols zu der Reaktionsmischung, die aus Dimethylsulfoxid, NaPNTSA und dem Uebergangsmetallkatalysator besteht, während gleichzeitig Luft durch die Mischung geleitet wird, erreicht man eine kleine aber reproduzierbare Ausbeutesteigerung um 3-5 % auf 96-98 %. Somit ist diese Reihenfolge der Zugabe bevorzugt.

Die Lösung des Alkalimetallhydroxides in Wasser oder einem $C_1$-$C_4$-Alkohol gibt man nach Möglichkeit während 5-45 Minuten, vorzugsweise 10-20 Minuten und besonders bevorzugt innerhalb ungefähr 15 Minuten zu der gekühlten stark durchmischten sauerstoffgesättigten Reaktionsmischung. Weitere 50-80 Minuten, vorzugsweise 60-70 Minuten Durchmischung währenddessen man Sauerstoff (Luft) durch die Mischung in ausreichendem Masse leitet genügen, um die Reaktion zu vervollständigen. Die Reaktion wird daraufhin beendet, dadurch dass man die überschüssige Base mit z.B. konzentrierter Schwefelsäure oder 20-25%igem Oleum neutralisiert.

C. DNS Isolierung

Die DNS kann auf verschiedenste Art und Weise aus der Reaktionsmischung als Dinatriumsalz isoliert werden. Bedingt durch die sehr hohe Reinheit und hohe Ausbeute ist es möglich, alles Dimethylsulfoxid, Wasser (und Alkohol) z.B. in einem Vakuumrotationsverdampfer bei ungefähr 200 °C abzuziehen um DNS hoher Qualität zu erhalten.

Eine andere Möglichkeit besteht darin, DNS aus der Dimethylsulfoxidlösung auszukristallisieren, indem man wässrige Kochsalzlösung dazugibt. Bei einer bevorzugten Variante dieses Vorgehens wird das Wasser, gegebenenfalls der Alkohol und 50-85 % des Dimethylsulfoxids durch Destillation entfernt. Die DNS wird daraufhin aus der konzentrierten Lösung durch Zugabe von Wasser und/oder Kochsalzlösung ausgefällt.

Weiterhin kann man die DNS aus der Dimethylsulfoxidreaktionsmischung durch die Zugabe einer grossen Menge eines organischen Stoffes, in welchem DNS praktisch unlöslich ist, zurückgewinnen. Es eignen sich aromatische Stoffe, wie z.B. Toluol. Vorteilhaft sind Alkohole, besonders jene Alkohole, die man zum Auflösen der Base benutzt. Als Alkohol kommt besonders Ethanol, einschliesslich der Mischungen mit Methanol und/oder Isopropanol in Frage. Normalerweise garantiert die zwei- bis dreifache Menge bezogen auf Dimethylsulfoxid des zweiten Stoffes eine hohe Ausbeute der DNS.

Der Feststoff kann auf an sich bekannte Art und Weise von der Flüssigkeit abgetrennt werden, wie z.B. durch Filtrieren oder Zentrifugieren.

Ein besonderer Vorteil bei Verwendung eines Mangansalzes, insbesondere $Mn(OAc)_2 \cdot 4H_2O$ als Uebergangsmetallkatalysator, in Verbindung mit Ethanol als Ausfällungsreagenz, liegt in der Tatsache, dass ungefähr 90 % der Manganverbindung in Lösung bleibt und somit für die nachfolgende Umsetzung zur Verfügung steht. Somit wird in einer besonders bevorzugten Ausführungsform die DNS aus der Reaktionsmischung nach der Neutralisierung durch Zugabe von Ethanol ausgefällt und durch Filtration abgetrennt. Das Filtrat, welches noch einwenig DNS und Nebenprodukte enthält, wird dann eingesetzt, um den feuchten Filterkuchen von NaPNTSA zu lösen. Das Ethanolwasserazeotrop wird daraufhin durch Vakuumdestillation entfernt und die darauf folgende Oxidation wird nur noch unter Verwendung von 10 % der normalen $Mn(OAc)_2 \cdot 4H_2O$ Menge durchgeführt.

Bedingt durch die hohe Ausbeute und die Möglichkeit, die Lösungsmittel zurückzugewinnen, sind die benötigten Abwasserbehandlungen wesentlich geringer als in den bisher beschriebenen Herstellungsverfahren.

Die folgenden Beispiele erläutern die Erfindung. Sie umfassen Varianten und allgemeine Durchführungsbereiche, ohne dass sich die Erfindung auf solche Bereiche beschränkt. Wenn nicht anders vermerkt, beziehen sich Prozentangaben auf Gewichtsprozente.

Beispiel 1: Darstellung von NaPNTSA mit Hilfe von $Na_2CO_3$

Man gibt in einen 1 l Erlenmeyerkolben 480 g Wasser und 48 g $Na_2CO_3$. Die Mischung wird auf 70 °C erhitzt und eine Roh-HPNTSA-Lösung wird, während die Temperatur oberhalb 70 °C gehalten wird, so zugegeben, dass man starkes Schäumen vermeidet. Die Zugabe von Roh-HPNTSA-Lösung (ca. 34%ig HPNTSA) wird fortgeführt bis die Reaktionsmasse einen pH-Wert von 8 aufweist. Die gerührte Reaktionsmischung wird auf Raumtemperatur abgekühlt und anschliessend vakuumfiltriert. Das Filtrat wird nochmals filtriert. Die vereinigten Filterkuchen werden 48 Stunden bei 60 °C und 250 Torr im Vakuum getrocknet. Die Ausbeute beträgt 86 % weisse NaPNTSA•$H_2O$ (Gehalt an trockener HPR/TSA 80-82 %). Weiteres Produkt kann aus dem Filtrat gewonnen werden.

Anstelle der Trocknung kann der nasse Filterkuchen wie in Beispiel 2 in DMSO gelöst werden.

Beispiel 2: Darstellung von NaPNTSA mit Hilfe von NaOH

In einen 1 l Fünfhalskolben mit eingebauten Schikanen, Heizmantel, Thermometer, Claisen-Kühler und regulierbarem Rührmotor gibt man 516,7 g zurückgeführtes NaPNTSA-Filtrat oder eine 21%ige $Na_2SO_4$-Lösung. Man erwärmt die Mischung auf 90 °C und gibt 33,7 g $Na_2SO_4$ dazu. Nachdem das $Na_2SO_4$ sich aufgelöst hat und die Reaktionsmischung auf 95 °C erhitzt worden ist, gibt man tropfenweise 250 g Roh-HPNTSA-Lösung (ca. 34%ig HPNTSA) mit Hilfe eines 250 ml Druckausgleichtropftrichters hinzu, wobei man während der Zugabe die Temperatur oberhalb 95 °C hält. Nach der Zugabe der HPNTSA-Lösung gibt man 25%ige NaOH (ungefähr 120 g) hinzu, bis der pH zwischen 3,6-6,0 ist (man kann auch 50%ige NaOH verwenden, wenn man das $H_2O/Na_2SO_4$-Verhältnis daran anpasst). Der Heizmantel wird entfernt und man lässt die Reaktionsmasse unter Rühren abkühlen. Die Kristallisation der NaPNTSA beginnt bei 85-90 °C. Nachdem die Reaktionsmasse auf 40-45 °C abgekühlt ist, benutzt man ein Eisbad um sie auf 20-27 °C abzukühlen. Nachdem man die Reaktionsmasse bei 20-27 °C während 30 Minuten hält, wird sie abfiltriert mit Hilfe eines 15 cm Büchnertrichters und Filtrierpapier der Firma Whatman (Nr. 541). Man zieht noch während einer Stunde nach dem Entfernen der Flüssigkeit Vakuum. Die Ausbeute bezogen auf HPNTSA beträgt 99,8 %.

Die in den folgenden Beispielen benutzte NaPNTSA/DMSO-Lösung wird hergestellt, indem man den feuchten NaPNTSA-Filterkuchen in DMSO löst und zur Entfernung des Wassers eine Vakuumdestillation bei 36 Torr durchführt. Wenn die Temperatur konstant bleibt, hat man einen Wassergehalt von $< 0,3$ % erreicht. Der feuchte Filterkuchen kann jedoch auch wie in Beispiel 1 beschrieben getrocknet werden.

Beispiel 3:

In ein 1 l zylindrisches Reaktionsgefäss, welches Schikanen eingebaut hat und mit einem Mantel, mit einer unteren Ausflussöffnung und einem Fünfhalsdeckel ausgestattet ist, gibt man 672,56 g einer getrockneten NaPNTSA/DMSO-Mischung (nach Beispiel 2, entsprechend 0,544 Mol NaPNTSA). Zu dieser Mischung gibt man 0,392 g (0,0016 Mol) $Mn(OAc)_2 \cdot 4H_2O$. Man rührt die Mischung mit einem mechanischen Rührer (700-750 Umdrehungen pro Minute) bis sich die Feststoffe aufgelöst haben. Man sättigt die Reaktionsmischung mit unterhalb des Rührers eingeblasener trockener Luft (Flussrate 900-1000 ml/Min.). Mit Hilfe eines Kältebades (Haake Modell A81) wird die Mischung auf 15 °C abgekühlt. Bei einer Temperatur von 15-18 °C und einem Luftstrom von 900-1000 ml pro Min. gibt man 47,8 ml (0,068 Mol, 0,125 Moläquivalente berechnet auf NaPNTSA) 5,7%ige NaOH in wässrig-alkoholischer Lösung (83,7 % Ethanol, 4,4 % Methanol, 4,9 % 2-Propanol, 6,9 % Wasser) über einen Zeitraum von 13,5 Min. hinzu mit Hilfe eines automatischen Titriergerätes (Metrohm Modell 655 Dosimat). Nach 70 Min. neutralisiert man die Reaktionsmasse mit 2,8 g 98%iger $H_2SO_4$. Man kann auch eine äquivalente Menge 20%iges Oleum verwenden. Die Ausbeute an DNS bestimmt mit HPLC beträgt 96,9 %.

Beispiel 4:

Beispiel 3 wird mit folgenden Mengen wiederholt: 653,2 g NaPNTSA/DMSO-Mischung (entsprechend 0.465 Mol NaPNTSA), 2,7 g (0,011 Mol) $Mn(OAc)_2 \cdot 4H_2O$. Die Luftflussrate beträgt 900 ml pro Minute und die Rührgeschwindigkeit 735 Umdrehungen pro Minute. Nachdem man die Reaktionsmasse auf 10°C abgekühlt hat, gibt man 52,5 g (0,074 Mol, 0,158 Moläquivalente berechnet auf NaPNTSA) 5,6%iger NaOH in wässrigem Alkohol über einen Zeitraum von 45 Minuten zu der Mischung, während man Luftstrom und Temperatur konstant hält. Nach 65 Minuten Reaktionszeit beträgt die Ausbeute an DNS, ermittelt durch HPLC 98,0 %.

Beispiele 5-8:

Die folgenden Beispiele erläutern den Einfluss unterschiedlicher Mengen an Manganacetat auf die Ausbeute.

Man wiederholt Beispiel 3 mit 653,0 g einer NaPNTSA/DMSO-Mischung (enthaltend 0,431 Mol NaPNTSA) und den unten angegebenen Mengen des $Mn(OAc)_2 \cdot 4H_2O$ Katalysators. Die Flussrate der Luft wird konstant gehalten bei 800-840 ml pro Minute.

In allen Beispielen wird die Reaktionsmasse auf 10-12°C abgekühlt bevor 52,5 ml (0,063 Mol, 0,145 Moläquivalente berechnet auf NaPNTSA) 5,6%ige NaOH in wässrigem Alkohol über einen Zeitraum von 15 Minuten zugegeben werden. Die Katalysatormengen, Reaktionszeiten und Ausbeuten bestimmt durch HPLC sind wie folgt:

| Beispiel Nr. | Katalysator (g, Mol) | Reaktionszeit (Min.) | Ausbeute in % DNS |
|---|---|---|---|
| 5 | 0,68 (0,0028) | 51 | 95,9 |
| 6 | 0,32 (0,0013) | 54 | 97,5 |
| 7 | 0,17 (0,0007) | 53 | 95,5 |
| 8 | 0,08 (0,0003) | 60 | 87,9 |

Beispiel 9:

Es wird das gleiche Reaktionsgefäss wie in Beispiel 3 verwendet. Man gibt in das Reaktionsgefäss: 66,5 g (0,253 Mol) NaPNTSA, 1,6 g (0,0065 Mol) $Mn(OAc)_2 \cdot 4H_2O$ Katalysator und 300,0 g trockenes DMSO. Die Mischung wird bei 720 Umdrehungen pro Minute solange gerührt, bis die Feststoffe gelöst sind. Die Lösung wird mit Luft gesättigt (Flussrate 550 ml pro Minute). Nachdem die Reaktionsmasse auf 11°C abgekühlt worden ist, wird über einen Zeitraum von 30 Minuten 99 ml (0,035 Mol, 0,137 Moläquivalente berechnet auf NaPNTSA) 1,8%ige NaOH in wasserfreiem 2-Propanol zugegeben. Nach 20 Minuten beträgt die Ausbeute an DNS 95,6 % (nach HPLC).

Beispiel 10:

Beispiel 3 wird wiederholt mit dem Unterschied, dass 651,8 g einer NaPNTSA/DMSO-Mischung, welche 0,444 Mol NaPNTSA enthält, in das Reaktionsgefäss gegeben werden, zusammen mit 1,8 g (0,0072 Mol) gemahlenem $CuSO_4 \cdot 5H_2O$ als Katalysator. Die Flussrate der Luft beträgt 930 ml pro Minute. Nachdem die Mischung auf 10°C abgekühlt worden ist, gibt man während 32 Minuten 52,5 ml (0,059 Mol, 0,133 Moläquivalente berechnet auf NaPNTSA) 5,3%iger NaOH in alkoholischer Lösung zu. Nach 70 Minuten beträgt die Ausbeute an DNS 95,4 % (nach HPLC).

Beispiel 11:

Beispiel 3 wird wiederholt, jedoch werden 623,4 g einer NaPNTSA/DMSO-Mischung (enthaltend 0,436 Mol NaPNTSA) zusammen mit 2,7 g (0,0108 Mol) $CuSO_4 \cdot 5H_2O$ in das Reaktionsgefäss gegeben. Die Flussrate der Luft beträgt 900 ml pro Minute und die Reaktionsmasse wird bei 750 Umdrehungen pro Minute gerührt während man sie auf 12°C abkühlt. Während 30 Minuten gibt man 173,4 ml (0,061 Mol,

0,139 Moläquivalente berechnet auf NaPNTSA) 1,8%ige NaOH in wasserfreiem 2-Propanol zu der Reaktionsmasse. Nach 50 Minuten beträgt die DNS-Ausbeute 91,08 % (nach HPLC).

Beispiele 12-17 zeigen, dass das erfindungsgemässe Verfahren auch mit mehr als der katalytischen Menge Base durchgeführt werden kann.

Beispiel 12:

In ein zylindrisches Reaktionsgefäss von 1 l ausgestattet mit einer unteren Ausflussöffnung und einem Fünfhalsdeckel gibt man 60,1 g (0,227 Mol) NaPNTSA, 320,0 g DMSO und 1,5 g (0,006 Mol) $CuSO_4 \cdot 5H_2O$. Die Reaktionsmischung wird mit einem mechanischen Rührer bei 715 Umdrehungen pro Minute gerührt, die Flussrate der unterhalb des Rührers eingedüsten Luft beträgt 550 ml pro Minute. Mit einem Eisbad wird die Lösung auf 14° C gekühlt. Während 30 Minuten gibt man tropfenweise mit Hilfe eines 125 ml Druckausgleichtropftrichters 10 g (0,25 Mol, 1,10 Moläquivalente berechnet auf NaPNTSA) NaOH hinzu, welche in 70,0 g Methanol gelöst worden ist. Während der Reaktion hält man die Temperatur der Reaktionsmasse unterhalb 15° C. Nach 60 Minuten beträgt die Ausbeute an DNS 95,6 % (nach HPLC).

Beispiel 13:

Beispiel 12 wird mit folgenden Mengen wiederholt: 65,0 g (0,246 Mol) NaPNTSA, 319,6 g DMSO und 1,1 g (0,0065 Mol) $MnSO_4 \cdot H_2O$. Nachdem die Mischung auf 14° C abgekühlt ist, gibt man während 30 Minuten 11 g (0,275 Mol, 1,12 Moläquivalente berechnet auf NaPNTSA) NaOH aufgelöst in 70 g Methanol zu dieser Mischung. Nach 90 Minuten bei 14° C beträgt die Ausbeute von DNS 85,8 % (nach HPLC).

Beispiel 14:

Beispiel 12 wird mit folgenden Mengen wiederholt: 65,0 g (0,246 Mol) NaPNTSA, 319,7 g DMSO und 1,7 g (0,0061 Mol) $FeSO_4 \cdot 7H_2O$. Nachdem die Mischung auf 16° C abgekühlt ist, gibt man während 30 Minuten 11 g (0,275 Mol, 1,12 Moläquivalente berechnet auf NaPNTSA) NaOH aufgelöst in 71,1 g Methanol zu dieser Mischung. Nach 60 Minuten beträgt die Ausbeute an DNS 90,9 % (nach HPLC).

Beispiel 15:

Beispiel 12 wird mit folgenden Mengen wiederholt: 69,4 g (0,262 Mol) NaPNTSA, 320,0 g DMSO und 1,5 g (0,0061 Mol) $Mn(OAc)_2 \cdot 4H_2O$. Nachdem die gut gerührte Mischung auf 13° C abgekühlt ist, gibt man während 45 Minuten 11,0 g (0,275 Mol, 1,05 Moläquivalente berechnet auf NaPNTSA) NaOH aufgelöst in 70 g Methanol zu dieser Mischung. Nach 75 Minuten beträgt die Ausbeute an DNS 95,4 % (nach HPLC).

Beispiel 16:

In ein 1 l Gefäss wie in Beispiel 3, wird 153,8 g DMSO, 10,0 g (0,25 Mol, 1,40 Moläquivalente berechnet auf NaPNTSA) NaOH, 1,5 g (0,0060 Mol) $CuSO_4 \cdot 5H_2O$, 5,5 g $H_2O$ und 2,5 g Tributylmethylammoniumchlorid gegeben. Die Flussrate der Luft beträgt 70-80 ml pro Minute. Nachdem die Reaktionsmasse auf 60° C erhitzt worden ist, gibt man tropfenweise während 15 Minuten mit Hilfe eines 500 ml Druckausgleichtropftrichters 244,4 g NaPNTSA/DMSO-Lösung (enthaltend 0,179 Mol NaPNTSA) zu dieser Mischung. Nach 65 Minuten beträgt die Ausbeute an DNS 74 % (nach HPLC).

Beispiel 17:

Dieses Beispiel zeigt, dass die Reaktion in Abwesenheit von etwas Wasser und/oder $C_1$-$C_4$-Alkohol nicht so gut verläuft.

Beispiel 3 wird mit folgenden Mengen wiederholt: 619,4 g einer NaPNTSA/DMSO-Mischung (enthaltend 0,528 Mol NaPNTSA), 0,38 g (0,0016 Mol) $Mn(OAc)_2 \cdot 4H_2O$. Die Flussrate der Luft beträgt 900-920 ml pro

Minute. Ueber einen Zeitraum von 5 Minuten gibt man zu dieser Mischung 57,1 g (0,139 Mol, 0,26 Moläquivalent berechnet auf NaPNTSA) einer NaOH/DMSO-Mischung, die man durch Mischen von 15,0 g NaOH und 140,0 g DMSO erhält. Nach 90 Minuten beträgt die Ausbeute an DNS nur 84,9 % (nach HPLC).

Beispiel 18:

Beispiel 3 wird mit folgenden Mengen wiederholt: 550,5 g einer NaPNTSA/DMSO-Mischung (enthaltend 0,469 Mol NaPNTSA) und 0,33 g (0,0013 Mol) $Mn(OAc)_2 \cdot 4H_2O$. Die Flussrate der Luft beträgt 1000 ml pro Minute. Die Mischung wird bei 1000 Umdrehungen pro Minute gerührt und nachdem die Feststoffe sich gelöst haben auf 20 °C abgekühlt. Bei einer Temperatur unterhalb 20 °C werden nun während 30 Minuten 3,32 ml (5,05 g, 0,063 Mol, 0,135 Moläquivalente berechnet auf NaPNTSA) 50%ige NaOH zugegeben. Nach 90 Minuten beträgt die Ausbeute an DNS nur 88,2 % (nach HPLC) wegen der schlechten Verteilung der Base in der Reaktionsmischung.

Beispiel 19:

Beispiel 18 wird mit folgenden Mengen wiederholt: 672,2 g einer NaPNTSA/DMSO-Mischung (enthaltend 0,553 Mol NaPNTSA), 0,38 g (0,0016 Mol) $Mn(OAc)_2 \cdot 4H_2O$. Die Flussrate der Luft beträgt 990 ml pro Minute. Die Mischung wird bei 1000-1040 Umdrehungen pro Minute gerührt und auf 17 °C abgekühlt. Während 25 Minuten werden 3,78 ml (5,75 g, 0,072 Mol, 0,13 Moläquivalente berechnet auf NaPNTSA) 50%ige NaOH gelöst in 5,0 g Methanol zu dieser Mischung gegeben. Die Ausbeute an DNS beträgt 94,1 % (nach HPLC) nach 70 Minuten.

Beispiele 20-24:

Beispiel 3 wird mit folgenden Mengen wiederholt: 655,0 g NaPNTSA/DMSO-Lösung (enthaltend 0,478 Mol NaPNTSA), 1,0 g (0,0041 Mol) $Mn(OAC)_2 \cdot 4H_2O$ und 28,5 g Wasser. Die Flussrate der Luft beträgt 1000 ml pro Minute und die Mischung wird bei 800 Umdrehungen pro Minute gerührt. Nachdem die Reaktionsmasse auf 13 °C abgekühlt worden ist wird innerhalb von 20 Minuten unter Beibehaltung der Luftzufuhr und des Rührens und bei einer Temperatur zwischen 15 und 18 °C eine Lösung von 11,4 g (0,143 Mol, 0,298 Moläquivalente berechnet auf NaPNTSA) wässriger 50%ige NaOH aufgelöst in unterschiedlichen Mengen Methanol zugegeben. Die Reaktion wird mit HPLC verfolgt und so die Zeit bis zur maximalen Ausbeute im Reaktor bestimmt. Die folgende Tabelle führt die Reaktionszeiten, Methanolmengen und DNS Ausbeuten (im Reaktor, durch HPLC) auf.

| Beispiel Nr. | MeOH (g) | Reaktionszeit (min.) | Ausbeute in % DNS |
|---|---|---|---|
| 20 | 40 | 60 | 96.1 |
| 21 | 30 | 60 | 94.9 |
| 22 | 20 | 90 | 93.2 |
| 23 | 10 | 90 | 91.7 |
| 24 | 0 | 90 | 90.1 |

Beispiele 25-26:

Beispiel 3 wird mit folgenden Mengen wiederholt: 657,0 g einer NaPNTSA/DMSO-Lösung (enthaltend 0.478 Mol NaPNTSA), 1,0 g $Mn(OAC)_2 \cdot 4H_2O$ und unterschiedliche Mengen Wasser. Die Flussrate der Luft beträgt 1000 ml pro Minute und die Mischung wird bei 800 Umdrehungen pro Minute gerührt. Nachdem die Reaktionsmasse auf 13 °C abgekühlt worden ist gibt man innerhalb von 20 Minuten bei konstantem Luftstrom und Rühren und bei einer Temperatur zwischen 15 und 18 °C eine Lösung von 5,7 g (0,143 Mol, 0,3 Moläquivalente berechnet auf NaPNTSA) NAOH aufgelöst in 40 g wasserfreiem Alkohol hinzu. Die Reaktion wird mit HPLC verfolgt und so die benötigte Zeit für die maximale Ausbeute im Reaktor bestimmt.

Die folgende Tabelle zeigt die erhaltenen Resultate.

| Beispiel Nr. | $H_2O$ (g) | Reaktionszeit (min.) | Ausbeute in % DNS |
|---|---|---|---|
| 25 | 28.8 | 60-90 | 96.2 |
| 26 | 61.3 | 90-120 | 97.4 |

Beispiel 27:

Beispiel 25-26 wird mit folgenden Mengen wiederholt: 655,7 g NaPNTSA/DMSO-Lösung (enthaltend 0,477 Mol NaPNTSA) und 135 g Wasser. Nach 265 Minuten wird die maximale Ausbeute von 89,5 % (nach HPLC) erreicht.

Beispiel 28:

Dieses Beispiel beschreibt die Oxidation in Abwesenheit eines $C_1$-$C_4$-Alkohols.

Beispiel 3 wird mit folgenden Mengen wiederholt: 688,9 g NaPNTSA/DMSO-Mischung (enthaltend 0,549 Mol NaPNTSA und 0,84 g (0,0041 Mol) $Mn(OAC)_2 \cdot 4H_2O$. Die Flussrate der Luft beträgt 1000 ml pro Minute und die Mischung wird bei 800 Umdrehungen pro Minute gerührt. Nachdem die Reaktionsmasse auf 12° C abgekühlt worden ist gibt man 43,6 g (0,218 Mol, 0,4 Moläquivalente berechnet auf NaPNTSA) 20%ige wässrige NAOH innerhalb 20 Minuten zu währenddessen man die Temperatur zwischen 15-19° C hält. Nach einer Reaktionszeit von 120 Minuten beträgt die Ausbeute an DNS 94,5 % (nach HPLC).

Beispiel 29:

Beispiel 3 wird mit folgenden Mengen wiederholt: 611,7 g NaPNTSA/DMSO-Mischung (enthaltend 0,436 Mol NaPNTSA). Das verwendete DMSO wurde in sechs vorhergehenden Reaktionen benutzt und durch Entfernen überschüssigen Alkohols und Wasser mit Hilfe einer Vakuumdestillation zurückgewonnen. Das so zurückgewonnene DMSO enthielt 2,3 % DNS, lösliche Verunreinigungen und $Mn(OAC)_2 \cdot 4H_2O$ (90 % der benötigten Menge) aus den vorangegangenen Reaktionen. Zu dieser Mischung gibt man 0,038 g frisches $Mn(OAC)_2 \cdot 4H_2O$ und die Luftzufuhr beträgt 900 ml pro Minute. Nachdem die stark berührte Reaktionsmasse auf 12° C abgekühlt ist gibt man innerhalb 20 Minuten 45,4 g (0,064 Mol, 0,146 Moläquivalente berechnet auf NaPNTSA) 5,6%ige NAOH (in wässrigem Alkohol) hinzu. Nach einer Reaktionszeit von 60 Minuten beträgt die um anfänglich in der DMSO-Lösung enthaltene Menge DNS korrigierte Ausbeute 95 % (nach HPLC). Die isolierte Ausbeute beträgt 95,6 % nach Aufarbeitung wie in Beispiel 32.

Beispiel 30:

Die Reaktionsmasse (723,7 g) aus Beispiel 3 wird in ein 6-Hals-2-liter-Mantelkristallisiergefäss überführt, welches eine untere Auslassöffnung, ein Thermometer, eine pH-Elektrode und einen mechanischen Rührer mit einem 4-Blattrührer besitzt. Bei einer Temperatur unterhalb 25° C wird die Reaktionsmasse innerhalb 10 Minuten durch die tropfenweise Zugabe von 2,8 g 98%ige $H_2SO_4$ (oder eine äquivalente Menge 23%igen Oleums) neutralisiert. Der pH-Wert der Lösung, welcher durch Zusammengeben von 4,3 g destilliertem $H_2O$ und 2,0 g neutralisierter Reaktionsmasse bestimmt wird, beträgt etwa 6,5. Daraufhin gibt man innerhalb 2-4 Minuten 507,9 g 94%igen Alkohols in dieses Gefäss. Nachdem man die Mischung 60 Minuten bei Raumtemperatur gerührt hat, wird sie auf 5° C mit einem Kühlbad abgekühlt und weitere 20 Minuten gerührt. Mit Hilfe eines Büchnerglasfilters wird die Reaktionsmasse im Vakuum filtriert, man erhält 956,0 g Filtrat. Der feuchte Filterkuchen wird dann mit 300,0 g 94%igem Alkohol (5° C) gewaschen, wobei man 320,5 g Filtrat erhält und 225,0 g Filterkuchen zurückbleibt, welcher ungefähr 95 % DNS enthält. Die isolierte Ausbeute and DNS beträgt 83 %. Die Filtrate enthalten ungefähr 12 % DNS und durch Rückführung der Lösungsmittel kann praktisch die gesamte Menge zurückgewonnen werden.

Beispiel 31:

Die Reaktinsmasse (461,7 g) wird wie in Beispiel 30 neutralisiert. Nach der Neutralisierung gibt man innerhalb 5 Minuten 732 g Toluol in das Reaktionsgefäss, um die DNS auszufällen. Der feuchte Filterkuchen wird durch Vakuumfiltration mit Hilfe eines Büchnerfilters (Whatman Nr. 1 Filterpapier) erhalten und wird im Vakuum (250 mm Hg) bei 75° C während 7 Stunden getrocknet. Die isolierte, trockene Ausbeute beträgt 83,6 % DNS aus einer Reaktionsmischung, welche nach HPLC-Analyse 88,5 % DNS enthielt.

Beispiel 32:

In ein 1 Liter Reaktionsgefäss wie in Beispiel 12 gibt man 69,4 g (0,262 Mol) NaPNTSA, 320 g DMSO, 20,6 g $NA_2SO_4$ und 1,5 g (0,0061 Mol) $Mn(OAC)_2 \cdot 4H_2O$. Die Reaktionsmischung wird bei 700 Umdrehungen pro Minute gerührt und bei einer Temperatur von 14° C mit Luft bei einer Flussrate von 550 ml pro Minute gesättigt. Daraufhin werden innerhalb 40 Minuten 11,0 g (0,275 Mol, 1,05 Moläquivalente berechnet auf NaPNTSA) NaOH aufgelöst in 70 g Methanol zugegeben. Nach 80 Minuten bei 14° C wird die Reaktionsmasse mit 16,2 g 23%igen Oleums neutralisiert. Die neutralisierte Reaktionsmasse (505,5 g) gibt man in einen 1-Liter Rundkolben welcher mit einem mechanischen Rührer, einem Rückflusskühler und einem Thermometer ausgerüstet ist. Die gerührte Reaktionsmischung wird auf 45° C erhitzt. Daraufhin gibt man eine wässrige Lösung von 40,6 g NaCl in 300 g Wasser zu der Mischung hinzu. Innerhalb 10 Minuten wird die Temperatur auf 75-80° C erhöht. Anschliessend wird die gerührte Mischung mit Hilfe eines Eisbades innerhalb von 50 Minuten auf 10° C abgekühlt. Die Masse wird im Vakuum abfiltriert mit Hilfe eines Büchnerfilters (Whatman Nr. 2 Filterpapier). Die isolierte Ausbeute beträgt 77,7 %, währenddessen die Ausbeute durch HPLC bestimmt 86,7 % ist.

Beispiel 33:

Die Reaktionsmasse (471,9 g) aus Beispiel 14 wird wie in Beispiel 32 neutralisiert und in einem Vakuumofen (74 mm Hg) bei 200° C 24 Stunden getrocknet. Die Reaktionsmasse wird von Zeit zu Zeit ausserhalb des Ofens gemahlen, um in den DNS-Kristallen eingeschlossenes Lösungsmittel freizusetzen. Das getrocknete Rohprodukt hat einen Gehalt von 88,5 % DNS, es finden sich keine Anzeichen für eine Zersetzung. Die Hauptverunreinigung ist $Na_2SO_4$ und dessen Hydratwasser.

Beispiel 34:

Beispiel 3 wird mit folgenden Mengen wiederholt: 672,2 g einer NaPNTSA/DMSO-Mischung (enthaltend 0,55 Mol NaPNTSA) und 0,394 g (0,0016 Mol) $Mn(OAc)_2 \cdot 4H_2O$. Die Flussrate der Luft beträgt 900-950 ml pro Minute und die Mischung wird bei 710-740 Umdrehungen pro Minute gerührt. Nachdem die Reaktionsmasse auf 10-12° C abgekühlt ist, gibt man innerhalb 25 Minuten bei konstanter Zufuhr von Luft und konstanter Temperatur 51,32 g (0,076 Mol, 0,138 Moläquivalente berechnet auf NaPNTSA) 5,9%iger NaOH in wässrigem SD3A Alkohol zu. Nach 65-75 Minuten Reaktionszeit beträgt die Ausbeute an DNS 95,38 % (nach HPLC). Die Reaktionsmasse (722,68 g) wird auf die gleiche Art wie in Beispiel 30 behandelt, mit der Ausnahme, dass man 539,08 g wässrigen SD3A Alkohol innerhalb 2-4 Minuten in das Reaktionsgefäss gibt, nachdem die Reaktionsmasse neutralisiert worden ist. Nachdem man die Reaktionsmasse 10 Minuten bei Raumtemperatur gerührt hat, wird sie auf 5° C mit einem Kühlbad gekühlt und weitere 15 Minuten gerührt. Die Mischung wird vakuumfiltriert mit Hilfe eines Glasbüchnerfilters, man erhält 1043,2 g Filtrat. Der feuchte Filterkuchen (177,9 g; 50,24 % DNS) wird anschliessend mit 444,75 g wässrigen SD3A Alkohol aufgeschlämmt und 30 Minuten unter Rückfluss erhitzt. Nach der Abkühlung auf Raumtemperatur wird die Mischung auf 5° C mit Hilfe eines Eis/Kochsalzbades gekühlt. Nach der Vakuumfiltration mit obengenanntem Filter erhält man 186,2 g feuchten Filterkuchen mit 50,79 Gew.% DNS (79,78 % isolierte DNS Ausbeute) und 405,5 g Filtrat.

Das Filtrat enthält ungefähr 16 % DNS. Durch die Aufarbeitung des Lösungsmittels, wie im nächsten Beispiel geschildert, kann diese DNS praktisch quantitativ zurückgewonnen werden.

Beispiele 35-40:

Beispiel 3 wird wiederholt jedoch werden sechs aufeinanderfolgende Reaktionen durchgeführt, wobei man das zurückgewonnene Dimethylsulfoxid der vorhergehenden Reaktion zusammen mit frischem Dimethylsulfoxid, welches den kleinen Anteil ersezt der durch die Wasser- und Alkoholentfernung verloren geht, verwendet. Für jede Reaktion wird eine Gesamtmenge von 540 g frischem und zurückgewonnenem Dimethylsulfoxid verwendet. Das Dimethylsulfoxid wird zurückgewonnen wie in Beispiel 30 beschrieben und enthält DNS, gelöste Verunreinigungen und $Mn(OAc)_2 \cdot 4H_2O$. Bei jeder Reaktion werden ungefähr 0,04 g $Mn(OAc)_2 \cdot 4H_2O$ zusätzlich zugesetzt. Die Reaktionsmischung wird bei 710-750 Umdrehungen pro Minute gerührt und auf 12° C abgekühlt die Flussrate der Luft beträgt 900-950 ml pro Minute. Innerhalb 23-27 Minuten gibt man in 48-56 g wässrigen SD3A Alkohols gelöstes NAOH hinzu. Nachdem die Reaktion vollständig ist, wird die Reaktionsmasse mit 2,8-3,7 g 98%iger $H_2SO_4$ neutralisiert. Wie in Beispiel 34 wird die DNS isoliert. Die Mengen und Ausbeuten für die sechs Reaktionen bei Verwendung von zurückgewonnenem Dimethylsulfoxid werden in der folgenden Tabelle aufgeführt. Die Mengenangaben für $Mn(OAc)_2 \cdot 4H_2O$ schliessen sowohl die Menge ein, die in dem zurückgewonnen Dimethylsulfoxid (ungefähr 90 % ermittelt durch Atomabsorptionsspektroskopie) enthalten ist, als auch die zusätzliche Menge, ungefähr 10 %, die man benötigt um die Verluste auszugleichen.

| Bsp.Nr. | NaPNTSA | NaOH | $Mn(OAC)_2 \cdot 4H_2O)$ | | Ausbeute | Ausbeute | Zeit |
|---|---|---|---|---|---|---|---|
| | (mol) | (mol) | (g) | (mol) | (HPLC) | (isoliert) | (min) |
| 35 | 0.535 | 0.072 | 0.3936 | 0.0016 | 98.2 | 92.1 | 60 |
| 36 | 0.558 | 0.085 | 0.4080 | 0.0017 | 95.0 | 95.5 | 83 |
| 37 | 0.581 | 0.103 | 0.3421 | 0.0014 | 95.0 | 95.8 | 100 |
| 38 | 0.540 | 0.094 | 0.4051 | 0.0017 | 95.2 | 101.8 | 125 |
| 39 | 0.529 | 0.088 | ca.0.4 | ca.0.0017 | 93.6 | 102.1 | 154 |
| 40 | 0.489 | 0.088 | 0.3691 | 0.0015 | 95.4 | 97.9 | 145 |

Beispiel 41:

Eine Reaktionsmasse wie aus Beispiel 3 wird in einem Rotationsverdampfer einer Vakuumdestillation bei 100° C und 6 mm Hg unterworfen, um Wasser, den Alkohol und ungefähr 80 % des DMSO zu entfernen. Die so erhaltene Masse enthält ungefähr 50 % DMSO und 50 % DNS. Um das DMSO zurückzugewinnen und die DNS zu isolieren, wird diese Aufschlämmung wie unten beschrieben zweimal umkristallisiert. Die Ausbeuteverluste an DNS werden niedrig gehalten 1 % der Theorie, dadurch dass man das Filtrat (ML2) und die Waschflüssigkeit (WW) der zweiten Kristallisation zurückführt.

Die Aufschlämmung (214,4 g) wird in ein 5-Hals-1-Liter-Rundgefäss gegeben, welches mit einer unteren Auslassöffnung und einem Rührer mit Teflonrührblatt versehen ist. Die Aufschlämmung wird durch Zugabe von 351,9 g des Filtrates einer zweiten Kristallisation (ML2) eines vorhergehenden Experimentes auflöst und dann durch eine äussere Heizung und bei langsamen Rühren auf 95° C erhitzt. Sobald die DNS aufgelöst ist, gibt man 30,0 g NaCl hinzu, um die Kristallisierung auszulösen. Nach 5 Minuten bei 95° C kühlt man die gerührte Kristallisationsmasse auf 5° C mit einem Eisbad ab. Vor der Filtration belässt man die Masse bei 5° C während 5 Minuten. Die Masse wird daraufhin mit Hilfe eines Polypropylenfiltertuches etwa 5 Minuten vakuumfiltriert.

Das Filtrat (354,0 g) der ersten Kristallisation (ML1) wird zur Rückgewinnung der verbliebenen 20 % DMSO destilliert. Man gibt den feuchten Filterkuchen (WC1), 239,7 g, in ein 1-Liter-Rundgefäss und löst ihn durch Zugabe von 360,6 g Waschflüssigkeit (WW) auf, die Waschflüssigkeit stammt von Nachwaschen eines feuchten Filterkuchens einer zweiten Kristallisation (WC2) eines vorhergehenden Experimentes mit Wasser. Durch Erwärmen auf 85° C mit Hilfe einer äusseren Heizung und bei langsamen Rühren wird der feuchte Filterkuchen (WC1) aufgelöst. Die gerührte Masse wird auf 5° C mit Hilfe eines Eisbades abgekühlt und mit Hilfe eines Polypropylenfiltertuches vakuumfiltriert. Die Filtration ist nach etwa 2 Minuten beendet. Das Filtrat (ML2), 351,9 g, kann für nachfolgende Kristallisationen der 50%igen Roh-DNS benutzt werden. Der feuchte Filterkuchen (WC2) der zweiten Kristallisation wird daraufhin mit 295 g kaltem (5° C) Wasser gewaschen. Nach der Zugabe des Waschwassers wird der Filterkuchen vakuumfiltriert und während total etwa 5 Minuten trocken gezogen. Die Waschflüssigkeit, 360,6 g wird für die zweite Kristallisation eines nachfolgenden Experimentes benutzt. Bei einer ausgedehnten Serie von Rückgewinnungsexperimenten hat der feuchte Filterkuchen am Schluss ein Gewicht von ungefähr 161 g mit einem Gehalt von 61,7 % DNS.

Dies entspricht einer isolierten Ausbeute von 94 % einer Reaktionsmischung welche einen Gehalt von 95 % (nach HPLC) aufweist.

## Ansprüche

1. Ein Verfahren zur Oxidation von 4-Nitrotoluol-2-sulfonsäure zu 4,4′-Dinitrostilben-2,2′-disulfonsäure dadurch gekennzeichnet, dass man langsam eine Lösung oder Dispersion einer katalytischen Menge eines Alkalimetallhydroxids oder -alkoxids zu einer Lösung eines Alkalimetallsalzes der 4-Nitrotoluol-2-sulfonsäure in Dimethylsulfoxid als Lösungsmittel, in Gegenwart einer katalytischen Menge eines organischen oder anorganischen Uebergangsmetallsalzes, -oxides oder -hydroxides gibt, wobei besagte Lösung mit Sauerstoff gesättigt wird, bis die Oxidation praktisch vollständig ist.

2. Ein Verfahren gemäss Anspruch 1, dadurch gekennzeichnet, dass man als Alkalimetallhydroxid Natriumhydroxid oder Natriummethoxid verwendet.

3. Ein Verfahren gemäss Anspruch 2, dadurch gekennzeichnet, dass das Alkalimetallhydroxid Natriumhydroxid ist.

4. Ein Verfahren gemäss Anspruch 1, dadurch gekennzeichnet, dass das Alkalimetallsalz der 4-Nitrotoluol-2-sulfonsäure das Natriumsalz ist.

5. Ein Verfahren gemäss Anspruch 3, dadurch gekennzeichnet, dass man Natriumhydroxid in Form einer Lösung in Wasser, in einem $C_1$-$C_4$-Alkohol oder deren Mischungen zugibt.

6. Ein Verfahren gemäss Anspruch 5, dadurch gekennzeichnet, dass man Natriumhydroxid in einem $C_1$-$C_4$-Alkohol löst, ausgewählt aus Methanol, Ethanol oder Isopropanol.

7. Ein Verfahren gemäss Anspruch 5, dadurch gekennzeichnet, dass man Natriumhydroxid als 10-50%ige wässrige Lösung zugibt.

8. Ein Verfahren gemäss Anspruch 1, dadurch gekennzeichnet, dass man 0,05-0,9 Moläquivalente Alkalimetallhydroxid oder -alkoxid verwendet.

9. Ein Verfahren gemäss Anspruch 6, dadurch gekennzeichnet, dass man 0,08-0,2 Moläquivalente Alkalimetallhydroxid oder -alkoxid verwendet.

10. Ein Verfahren gemäss Anspruch 1, dadurch gekennzeichnet, dass der Uebergangsmetallkatalysator ein anorganisches oder organisches Salz ausgewählt aus Magnesium, Kupfer oder Eisen ist.

11. Ein Verfahren gemäss Anspruch 10, dadurch gekennzeichnet, dass der Katalysator ein anorganisches oder organisches Magnesiumsalz ist.

12. Ein Verfahren gemäss Anspruch 10, dadurch gekennzeichnet, dass der Katalysator $Mn(OAc)_2 \cdot 4H_2O$ ist.

13. Ein Verfahren gemäss Anspruch 1, dadurch gekennzeichnet, dass die Oxidation bei erhöhtem Druck durchgeführt wird.

14. Ein Verfahren gemäss Anspruch 1, dadurch gekennzeichnet, dass die Oxidation bei einer Temperatur zwischen 8 und 60 °C durchgeführt wird.

15. Ein Verfahren gemäss Anspruch 14, dadurch gekennzeichnet, dass die Oxidation bei einer Temperatur zwischen 10 und 25 °C durchgeführt wird.

16. Ein Verfahren gemäss Anspruch 14, dadurch gekennzeichnet, dass die Oxidation bei einer Temperatur zwischen 12 und 20 °C durchgeführt wird.

17. Ein Verfahren gemäss Anspruch 1, dadurch gekennzeichnet, dass man das Alkalimetallhydroxid oder -alkoxid, 50-80 Minuten nachdem alle nicht gasförmigen Reaktanden zusammengegeben worden sind, neutralisiert.

18. Ein Verfahren gemäss Anspruch 1, dadurch gekennzeichnet, dass man die Alkalimetallhydroxid- oder -alkoxidlösung langsam innerhalb 5-40 Minuten zugibt.

19. Ein Verfahren gemäss Anspruch 18, dadurch gekennzeichnet, dass man die Natriumhydroxidlösung innerhalb 10-20 Minuten zugibt.

20. Ein Verfahren gemäss Anspruch 18, dadurch gekennzeichnet, dass man eine wässrige Natriumhydroxidlösung in einem $C_1$-$C_4$-Alkohol innerhalb 5-40 Minuten zugibt.

21. Ein Verfahren gemäss Anspruch 1, dadurch gekennzeichnet, dass die Sauerstoffquelle trockene Luft ist.

22. Ein Verfahren gemäss Anspruch 6, dadurch gekennzeichnet, dass man die Natriumhydroxidlösung in einem Alkohol, ausgewählt aus Methanol, Ethanol oder Isopropanol, zu einer sauerstoffgesättigten Mischung der Reaktanden gibt, wobei besagte Mischung der Reaktanden eine Temperatur zwischen 10 und 25 °C aufweist und dass man das Natriumhydroxid 50-80 Minuten nachdem alle nicht gasförmigen Reaktanden zusammen gegeben worden sind neutralisiert.

23. Ein Verfahren gemäss Anspruch 6, dadurch gekennzeichnet, dass das verwendete Dimethylsulfoxid das Filtrat einer vorangegangenen Herstellung von 4,4′-Dinitrostilben-2,2′-disulfonsäure ist, aus welchem Wasser und Alkohol durch Vakuumdestillation entfernt worden sind.

24. Ein Verfahren gemäss Anspruch 22, dadurch gekennzeichnet, dass nach der Neutralisation des Natriumhydroxids Wasser, Alkohol und Lösungsmittel durch Vakuumdestillation entfernt werden.

25. Ein Verfahren gemäss Anspruch 22, dadurch gekennzeichnet, dass weitere Schritte darin bestehen, dass man eine Flüssigkeit in welcher das Produkt schwer löslich ist zugibt und dass das ausgefällte Produkt aus dem Lösungsmittelgemisch abgetrennt wird.

26. Ein Verfahren gemäss Anspruch 22, dadurch gekennzeichnet, dass die Flüssigkeit in der das Produkt schwer löslich ist Ethanol oder Toluol ist.

27. Ein Verfahren gemäss Anspruch 25, dadurch gekennzeichnet, dass das Produkt durch Vakuumfiltration oder Zentrifugieren abgetrennt wird.

28. Ein Verfahren gemäss Anspruch 24, dadurch gekennzeichnet, dass nach der Neutralisierung des Natriumhydroxids 50-85 % des Dimethylsulfoxids durch Vakuumdestillation entfernt wird und das Produkt durch Zugabe einer wässrigen Salzlösung ausgefällt wird.